# EUROPEAN PATENT SPECIFICATION

(11) **EP 2 618 154 B1**
(45) Date of publication and mention of the grant of the patent: **14.12.2016**
(21) Application number: 11824725.3
(22) Date of filing: 26.08.2011
(51) Int. Cl.: G01N 33/574, G01N 21/64, G01N 21/78, G01N 33/536, G01N 33/58, G01N 33/68

(54) **METHOD FOR DETERMINING EFFECTIVENESS OF MEDICINE CONTAINING ANTIBODY AS COMPONENT**
VERFAHREN ZUR BESTIMMUNG DER EFFEKTIVITÄT EINER MEDIZIN MIT EINEM ANTIKÖRPER ALS BESTANDTEIL
MÉTHODE DE DÉTERMINATION DE L'EFFICACITÉ D'UN MÉDICAMENT CONTENANT UN ANTICORPS COMME COMPOSANT

(30) Priority: 17.09.2010 JP 2010209322
(43) Date of publication of application: 24.07.2013
(73) Proprietor: Tohoku University, Aoba-ku Sendai-shi Miyagi 980-8577 (JP)
(72) Inventor: MIYASHITA, Minoru, Sendai-shi Miyagi 980-8577 (JP); GONDA, Kohsuke, Sendai-shi Miyagi 980-8577 (JP); OHUCHI, Noriaki, Sendai-shi Miyagi 980-8577 (JP); TAKEDA, Motohiro, Sendai-shi Miyagi 980-8577 (JP)
(74) Representative: Appleyard Lees IP LLP
(86) International application number: PCT/JP2011/004763
(87) International publication number: WO 2012/035705

(56) References cited:
- EP-A1- 2 613 151
- WO-A1-2010/023917
- WO-A1-2010/084720
- JP-A- 8 334 466
- JP-A- 2006 506 943
- P. CONSTANTINOU ET AL: "Extending immunofluorescence detection limits in whole paraffin-embedded formalin fixed tissues using hyperspectral confocal fluorescence imaging", JOURNAL OF MICROSCOPY, vol. 234, no. 2, 1 May 2009 (2009-05-01), pages 137-146, XP055107055, ISSN: 0022-2720, DOI: 10.1111/j.1365-2818.2009.03155.x
- C. H. VAN DE LEST ET AL: "Elimination of autofluorescence in immunofluorescence microscopy with digital image processing.", JOURNAL OF HISTOCHEMISTRY & CYTOCHEMISTRY, vol. 43, no. 7, 1 July 1995 (1995-07-01), pages 727-730, XP055107062, ISSN: 0022-1554, DOI: 10.1177/43.7.7608528
- YUKIKO OTSUKA ET AL.: 'Formalin Kotei Paraffin Homai Hyohon kara Doko made Idenshi Kensaku wa Kano ka? Nyugan ni Okeru HER-2 Idenshi Ijo no FISH Kaiseki -Yuyosei to Chuiten, Men'eki Soshiki Kagaku tono Hikaku' CLINICAL TESTING vol. 50, no. 7, 2006, pages 753 - 760, XP008168376
- LEBEAU, ANNETTE ET AL.: 'HER-2/neu analysis in archival tissue samples of human breast cancer: Comparison of immunohistochemistry and fluorescence in situ hybridization' J CLIN ONCOL vol. 19, no. 2, 2001, pages 354 - 363, XP008163075
- TOMONOBU WATANABE: 'Kyoshoten Laser Kenbi System o Mochiita in vivo, in vitro ni Okeru Saibonai Shingo Dentatsu Keisoku Shuho no Kakuritsu ni Kansuru Kenkyu' SEITAI CHO BISAI ICHI BUNSHI KASHIKA GIJUTSU NI YORU NANO DDS TO GAN HYOTEKI CHIRYO NI KANSURU KENKYU, HEISEI 18 NENDO SOKATSU · BUNTAN KENKYU NENDO SHURYO HOKOKUSHO 2007, pages 15 - 17, XP001539090
- LEE, DONG KWAN ET AL.: 'Preparation of near- infrared quantum dots-herceptin conjugates for cancer imaging' MACROMOLECULAR RESEARCH vol. 18, no. 7, July 2010, pages 641 - 647, XP008163077
- TAKEO ITO: 'Soshiki Saibo Kagaku 2006 -Idenshi . Bunshi kara Saibo . Soshiki eno Kaiki-, I. Men'eki Soshiki - Saibo Kagaku -Kiso kara Oyo- Keiko Kotai no Aratana Tenkai -Nano Crystal Men'eki Keiko Kotaiho' SOSHIKI SAIBO KAGAKU vol. 2006, 2006, pages 25 - 34, XP008168872
- MAURIZIO SCALTRITI ET AL.: 'Expression of p95HER2, a Truncated Form of the HER2 Receptor, and Responces to Anti-HER2 Therapies in Breast Cancer' JNCI vol. 99, no. 8, 2007, pages 628 - 638, XP002540506

## Description

### Technical Field

The present invention relates to a method for determining effectiveness of a medicine containing an antibody as a component by employing a tissue staining method in which highly accurate and quantitative analysis can be conducted with influence of autofluorescence effectively removed.

### Background Art

Cancer is a disease that is one of the two main causes of death of adults together with vascular diseases such as cardiac infarction and brain infarction. An incidence rate of, for example, breast cancer is lower in Japan than in Western countries but recently shows an increasing tendency, and overtook the incidence rate of stomach cancer and came top of the incidence rate among women in 1998. According to a recent report of statistics of Ministry of Health, Labour and Welfare in 2005, the annual number of breast cancer cases is over 50000. The number is increasing year by year also in the whole world, and according to a WHO report in 2008, breast cancer is a disease of the top incidence rate among men and women, the annual number of cases exceeds 1380000, and it occupies approximately 23% of all cancers among women.

For diagnosis of cancer, in addition to diagnostic imaging such as X-ray CT and MRI, a method for detecting a cancer marker specifically expressed in specific cancer or a cancer marker leaking into blood or tissues is also employed. As a general screening test for breast cancer, interview, palpation, soft X-ray breast tomography (mammography), ultrasonography and the like are conducted, and when there is clinical suspicion, cytological diagnosis and biopsy are conducted so as to determine through pathological diagnosis whether or not the patient has cancer. The pathological diagnosis is significant for treatment or determination of prognosis of cancer, and an "HE (hematoxylin eosin) staining method for morphological observation" and an "immunohistochemistry method using an antibody against a cancer marker factor" are the core of this diagnosis. Due to recent appearance of antibody medicines in particular, immunohistochemistry has become very important.

HER2 protein, which is a gene product of human cancer gene HER2/neu (c-erbB-2), is a growth factor receptor belonging to the human epidermal growth factor receptor family and is transmembrane protein with a molecular weight of approximately 185 kDa having a tyrosine kinase active site on the cytoplasm side. In some human breast cancer cells, it is found that HER2 is highly expressed. Trastuzumab (generic name) commercially available as Herceptin (registered trademark), which is an antibody medicine containing an anti-HER2 antibody targeting human epidermal growth factor receptor 2 (HER2), a factor involved in growth of cancer, is known as a representative anticancer agent for breast cancer. Trastuzumab first specifically binds to HER2 and then exhibits an antitumor effect through antibody dependent cellular cytotoxicity (ADCC) using NK cells and monocytes as cells of action. As a method for determining effectiveness of administration of this agent, an immunohistochemistry (IHC) method for analyzing expression of protein such as HER2 protein and a FISH (fluorescence in situ hybridization) method for analyzing increase of the gene copy number of HER2 gene or the like are widely clinically employed. In the IHC method, an anti-HER2 antibody bound to a HER2 antigenic site is visualized by staining it with DAB (diaminobenzidine), so that the expression level of HER2 can be detected. The determination is, however, made based on rough criteria in merely four stages of Score 0 to 3 of the staining level. Therefore, this method is less quantitative and the criteria are varied depending upon the experience and skill of a pathologist, which has become a problem in the clinical setting. On the other hand, the FISH method is performed by using a probe for detecting HER2 gene and a probe for detecting the chromosome 17 centromere, and on the basis of the gene copy number of HER2 per chromosome 17 analyzed by the FISH method, it can be determined whether or not the HER2 gene copy number on the genome is increased. Although the FISH method is a quantitative test method, it cannot directly evaluate the amount of HER2 protein or the intracellular localization of HER2 protein. It is considered significant, for the treatment of cancer, to quantitatively analyze the "copy number of cancer gene" and the "amount of cancer gene product" in a localized site so as to examine the correlation therebetween. Due to such circumstances, it is necessary to develop a determination method in which the amount of cancer gene product in a localized site, namely, the amount of cancer-associated protein such as HER2 protein, can be quantitatively detected.

Recently, instead of an enzymic method using DAB, use of fluorescent particles of a quantum dot and the like attracts attention as a detection method excellent in detection sensitivity and quantitativeness. In the quantum dot, there is a proportional relationship between the number of particles and fluorescence brightness, and therefore, if a probe obtained by binding a quantum dot to an antibody is used in the immunohistochemistry method, there is high possibility that the amount of cancer marker protein may be highly accurately and quantitatively analyzed.

Furthermore, the response rate of single administration of trastuzumab in breast cancer patients is 15 to 34%, and such a low effect is also a problem as an antibody medicine. It is reported that although overexpression of HER2 is observed by immunohistostaining using a DAB method, the extracellular domain of HER2 recognized by trastuzumab is deleted in a patient in which a therapeutic effect attained by trastuzumab is low (Nahta R. et al., Breast Cancer Res. 2006; 8:215-223; and Scaltriti M. et al., J. Natl. Cancer Inst. 2007; 99:628-638). One of the causes of this problem seems that an antigenic site of HER2 recognized by an anti-HER2 antibody used in detection of HER2 in the diagnosis of breast cancer is different from an antigenic site of HER2 recognized by trastuzumab in the treatment of breast cancer.

### Prior Art Documents

### Patent Documents

EP2613151A1 relates to methods for determining cancer onset or cancer onset risk using quantitative tissue-staining.

### Non-patent Documents

Non-patent Document 1: Nahta R. et al., Breast Cancer Res. 2006; 8:215-223
Non-patent Document 2: Scaltriti M. et al., J. Natl. Cancer Inst. 2007; 99:628-638
Non-patent Document 3: Constantinou, et al., J. Microsc. 2009; 234:137-146, relates to high resolution microscopy of *ex vivo* tissues
Non-patent Document 4: Van de Lest, et al., J. Histochem. Cytochem 1995; 43;727-730, relates to the elimination of autofluorescence in immunofluorescence microscopy with digital image processing.

### Summary of the Invention

### Object to be Solved by the Invention

Since the fluorescent particles of a quantum dot and the like show their power in stability of light emitted by themselves and quantitative analysis, they are expected as a novel tool to be used in immunofluorescence staining, but their use for the quantitative analysis is limited to cultured cells alone at the present. This is principally because cultured cells show merely weak autofluorescence and hence the fluorescence brightness of quantum dot fluorescent particles can be calculated as a sufficiently high S (signal)/N (noise) ratio while biological tissues show very strong autofluorescence as compared with cultured cells, and hence the fluorescence brightness of the quantum dot fluorescent particles cannot be calculated as a sufficiently high S/N ratio. Although the amount of long wavelength autofluorescence of biological tissues is smaller than the amount of short wavelength autofluorescence, the influence of the autofluorescence cannot be ignored in observation, and hence, the quantitative analysis is difficult. Furthermore, since an antibody used for detecting HER2 protein in the diagnosis of breast cancer is different, in an antigenic determinant (epitope), from a therapeutic agent (an antibody) targeting HER2 protein, it is regarded that the current IHC method using an anti-HER2 antibody is insufficient as a method for selecting patients eligible for administration of trastuzumab in which HER2 including an antigenic determinant of trastuzumab is overexpressed. An object of the present invention is to highly accurately quantitatively determine, through a quantitative tissue staining method for biological tissues, protein recognized by an antibody used as an active ingredient of an antibody medicine such as trastuzumab or an antibody used for targeting a target site of the active ingredient, thereby providing a method for determining therapeutic effectiveness of a medicine containing such an antibody as a component.

### Means to Solve the Object

The present inventors considered that patients eligible for administration of trastuzumab can be more accurately selected by conducting a biological tissue staining method using trastuzumab directly labeled with quantum dot fluorescent particles. While earnestly examining a method for removing the influence of autofluorescence in the biological tissue staining method, the present inventors found the following: a breast cancer tissue sample immunostained with an anti-HER2 antibody, that is, an active ingredient of trastuzumab, labeled with quantum dot fluorescent particles (Qdot 705) (trastuzumab + QD705) is irradiated with excitation light of a wavelength of 488 nm, so as to obtain a fluorescence image of the quantum dot fluorescent particles (fluorescence of the quantum dot fluorescent particles + autofluorescence) by using a band-pass filter having a pass range of a fluorescence wavelength of 695 to 740 nm. Thereafter, an autofluorescence image including the autofluorescence alone is obtained on exactly the same focal surface and in the same field of view as in the fluorescence image by using a band-pass filter having a pass range of a fluorescence wavelength of 640 to 690 nm. When the fluorescence brightness of respective pixels of the autofluorescence image is subtracted from the fluorescence brightness of corresponding pixels of the fluorescence still image, an image (a corrected fluorescence image) including the fluorescence of the quantum dot fluorescent particles alone can be obtained with the autofluorescence image (autofluorescence) subtracted from the fluorescence still image. Furthermore, in order to specify the number of quantum dot fluorescent particles included in the corrected fluorescence image, a "blinking property" peculiar to the quantum dot was utilized. Specifically, since a single quantum dot fluorescent particle is in an off-state (a dark state) for approximately 4 seconds through excitation light irradiation of 20 seconds, average brightness of particles put in an off-state (a dark state) of approximately 4 seconds was calculated as fluorescence brightness per quantum dot fluorescent particle, and it was thus confirmed that the number of quantum dot fluorescent particles per cell in the corrected fluorescence image can be calculated. Moreover, it was also confirmed that the expression level of HER2 protein may be quantitatively determined in a wider range than in an IHC-DAB method by calculating the number of quantum dot fluorescent particles detected by using trastuzumab + QD705 in a mammary gland tissue of a breast cancer patient. In addition, it was found that there is correlation between the number of quantum dot fluorescent particles detected by using trastuzumab + QD705 and a therapeutic effect attained by trastuzumab. The present invention was accomplished on the basis of these findings.

Specifically, the present invention relates to: (1) a method for determining effectiveness of a medicine containing an antibody as a component by employing a tissue staining method comprising the steps of: (a) labeling an antibody in the medicine containing an antibody as a component with a fluorescent material and contacting the fluorescence-labeled antibody with a tissue sample; (b) obtaining a fluorescence image by irradiating, with excitation light, a tissue site contacted with the antibody; (c) obtaining an autofluorescence image in the same field of view and at the same focus as in the fluorescence image in a close region on a shorter wavelength side or a longer wavelength side of an acquisition wavelength region of fluorescence emitted by the fluorescent material; and (d) obtaining a corrected fluorescence image by performing image processing for removing fluorescence brightness of the autofluorescence image from fluorescence brightness of the fluorescence image, (2) the method according to (1), wherein the antibody in the medicine containing an antibody as a component is an antibody recognizing a growth regulatory factor or a metastasis regulatory factor of cancer, (3) the method according to (2), wherein the growth regulatory factor or the metastasis regulatory factor of cancer is human epidermal growth factor receptor 2 (HER2), (4) The method according to (2) or (3), wherein the cancer is breast cancer, (5) the method according to any one of (1) to (4), wherein a difference in wavelength between the acquisition wavelength region of fluorescence emitted by the fluorescent material and the close region is 100 nm or less, (6) the method according to any one of (1) to (5), wherein the fluorescent material is a fluorescent particle, (7) the method according to (6), wherein the fluorescent particle is a quantum dot fluorescent particle, (8) the method according to (6) or (7), further comprising the steps of: (e) counting a number of cells in the tissue site contacted with the antibody; (f) identifying a single fluorescent particle on the basis of the fluorescence image and measuring average fluorescence brightness per fluorescent particle within the corrected fluorescence image corresponding to the single fluorescent particle; and (g) obtaining a number of fluorescent particles by dividing total fluorescence brightness within the corrected fluorescence image by the average fluorescence brightness per fluorescent particle, and calculating a number of fluorescent particles per cell by dividing the number of fluorescent particles by the number of cells counted in step (e), and (9) the method according to (8), wherein the single fluorescent particle is specified by utilizing a blinking property of the fluorescent particle.

Furthermore, the present invention may be carried out using a system for determining effectiveness of a medicine containing an antibody as a component, comprising (A) an antibody in the medicine containing an antibody as a component, the antibody being labeled with a fluorescent material; (B) excitation light irradiation means; (C) fluorescence image obtaining means; and (D) a band-pass filter for obtaining a fluorescence image, wherein the antibody in the medicine containing an antibody as a component may be an antibody recognizing a growth regulatory factor or a metastasis regulatory factor of cancer, wherein the growth regulatory factor or the metastasis regulatory factor of cancer may be human epidermal growth factor receptor 2 (HER2). The system may further comprise (E) a band-pass filter for obtaining a cell nucleus fluorescence image, wherein the fluorescent material may be a fluorescent particle, and wherein the fluorescent particle may be a quantum dot fluorescent particle.

### Effect of the Invention

According to the present invention, the expression level of HER2 protein can be quantitatively determined by a tissue staining method using, for example, trastuzumab labeled with quantum dot fluorescent particles, with the influence of autofluorescence removed, in a wider range than in the tissue staining method (the IHC-DAB method) employed as one of conventional diagnosis methods for breast cancer, so that novel quantitative pathological diagnosis for breast cancer with high sensitivity can be performed instead of the IHC-DAB method. Furthermore, since it has been found that there is correlation between a detection level for HER2 protein attained by an anti-HER2 antibody, an active ingredient of trastuzumab, labeled with quantum dot fluorescent particles and a therapeutic effect attained by trastuzumab, it can be expected that the therapeutic effect attained by trastuzumab can be estimated by the present invention. In this manner, according to the present invention, effectiveness of not only trastuzumab but also medicines containing an antibody as a component in general can be determined. Moreover, when the present method for determining effectiveness of a medicine containing an antibody as a component is combined with any of the conventional techniques such as the FISH method, the gene copy number of HER2 and the amount of HER2 protein in breast cancer can be both quantitatively evaluated, and thus, the present invention is effective for conducting cancer treatment for breast cancer.

### Brief Description of Drawings

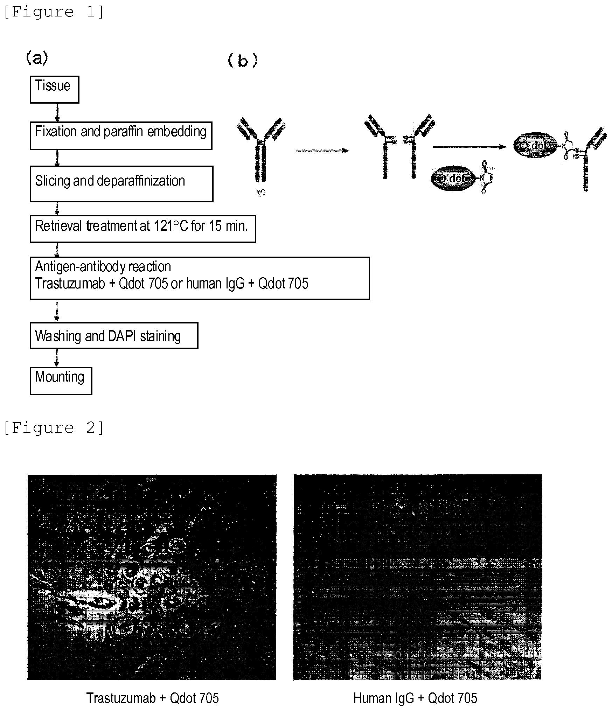

[Figure 1] Figure 1(a) is a diagram illustrating preparation of a tissue sample by employing an immunostaining method using quantum dot fluorescent particles (an IHC-QDs method). Figure 1(b) is a diagram illustrating a coupling reaction between a thiol group of an antibody (trastuzumab) and a maleimide group of the quantum dot fluorescent particle.
[Figure 2] Figure 2 illustrates representative examples of obtained fluorescence images.

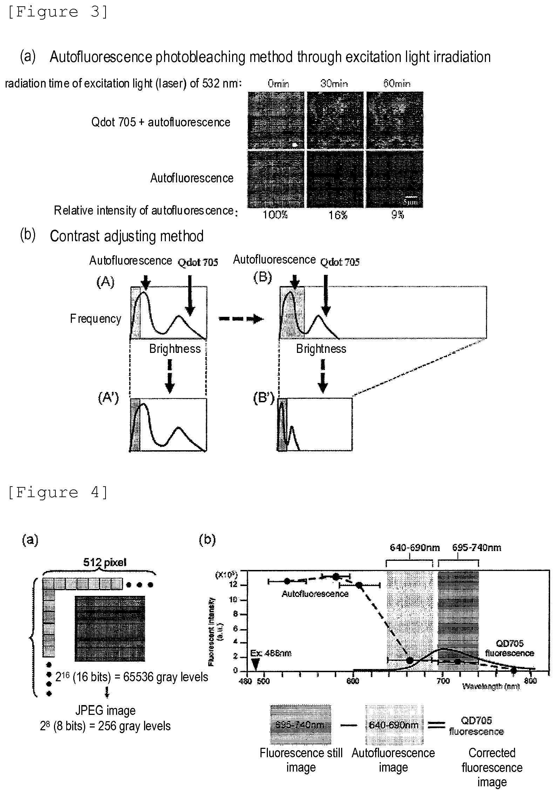

[Figure 3] Figure 3 is a diagram illustrating a conventional method for removing influence of autofluorescence. Figure 3(a) illustrates a result of an autofluorescence fading method with excitation light irradiation (see Hikage et al., Nanotechnology (2010)), and Figure 3 (b) is an explanatory diagram of a contrast adjusting method, in which a gray region corresponds to a range recognized as black to human eyes.
[Figure 4] Figure 4(a) is a diagram illustrating image conversion performed in image processing by subtraction. Figure 4(b) is a diagram illustrating fluorescence wavelength characteristics of quantum dot fluorescent particles and fluorescence wavelength characteristics of autofluorescence. A solid line corresponds to the fluorescence wavelength characteristics of quantum dot fluorescent particles (QD705). Respective error bar plots correspond to actually measured values of fluorescence brightness of autofluorescence on tissue sample sections obtained by using six kinds of band-pass filters respectively having pass ranges of fluorescence wavelengths of 505 to 545 nm, 565 to 595 nm, 585 to 630 nm, 640 to 690 nm, 695 to 740 nm and 760 to 800 nm. Average value plots of these actually measured values are connected with one another by a dotted line so as to be shown as the fluorescence wavelength characteristics of the autofluorescence. In addition, the wavelength widths of the band-pass filters are indicated by using bars.

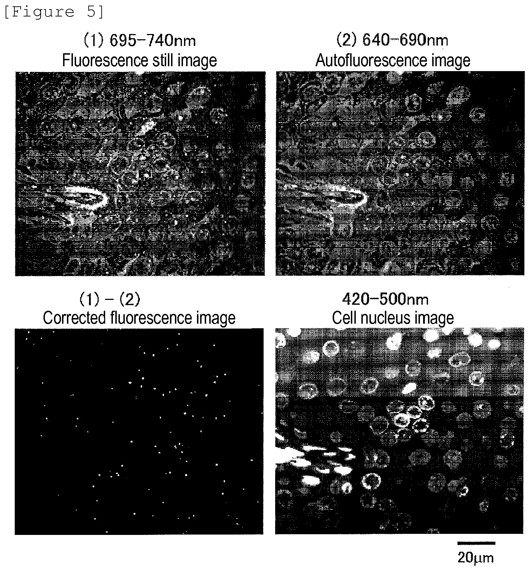

[Figure 5] Figure 5 is a diagram illustrating a method for generating a corrected fluorescence image with autofluorescence removed.

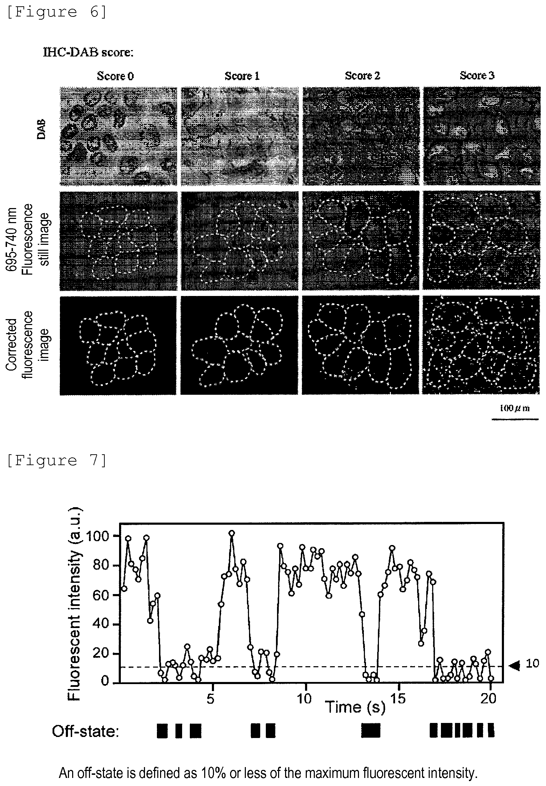

[Figure 6] Figure 6 is a diagram illustrating results of staining, by the IHC-DAB method and the IHC-QDs method, tissue samples having been subjected to the pathological diagnosis by the IHC-DAB method.
[Figure 7] Figure 7 is a diagram illustrating a blinking property of quantum dot fluorescent particles. The ordinate indicates fluorescence brightness of the quantum dot fluorescent particles, and the abscissa indicates irradiation time (sec.) for irradiating the fluorescence quantum dot particles with excitation light. An arrowhead indicates that 10% of the maximum fluorescence brightness of the quantum dot fluorescent particles is set as a threshold value of the fluorescence brightness.

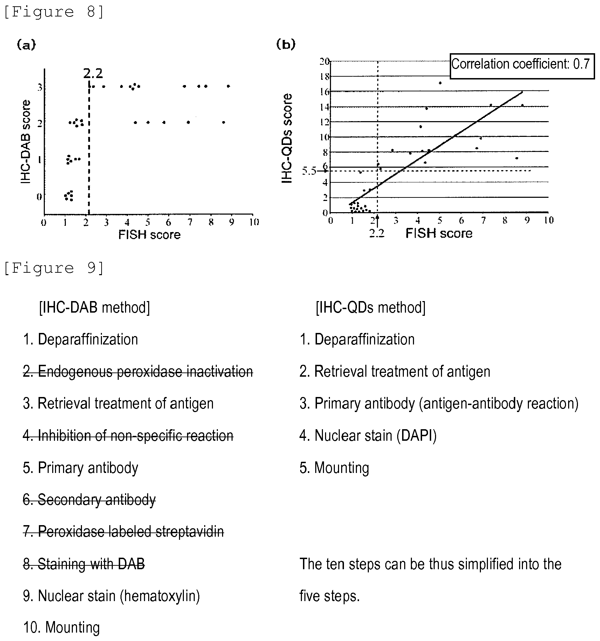

[Figure 8] Figure 8(a) is a diagram illustrating the relationship between the HER2 gene copy number calculated by the FISH method and the amount of HER2 protein calculated by the IHC-DAB method, where FISH score 2.2 corresponds to a threshold value of the FISH score for making pathological determination. Figure 8(b) is a diagram illustrating the relationship between the HER2 gene copy number calculated by the FISH method and the amount of HER2 protein calculated by the IHC-QDs method, where FISH score 2.2 and IHC-QDs score 5.5 respectively correspond to threshold values of the FISH score and the IHC-QDs score for making pathological determination.
[Figure 9] Figure 9 is a diagram illustrating an operation procedure of the IHC-DAB method and an operation procedure of the IHC-QDs method.

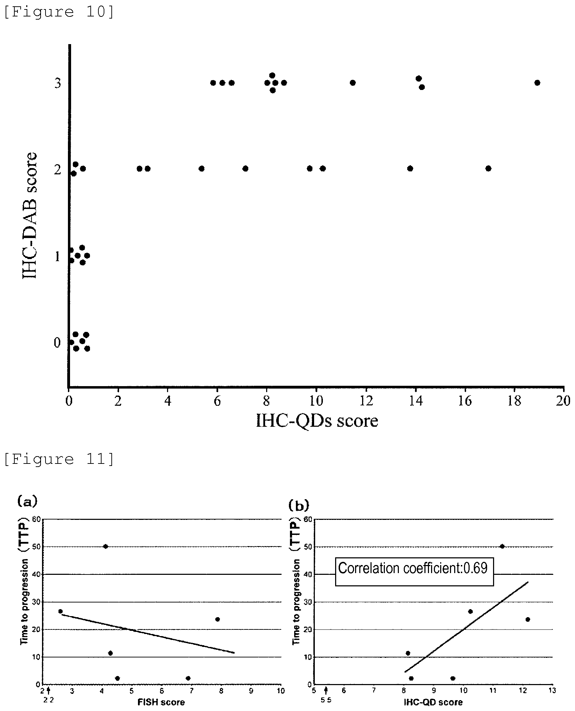

[Figure 10] Figure 10 is a diagram illustrating the relationship between the amount of HER2 protein calculated by the IHC-QDs method and the amount of HER2 protein calculated by the IHC-DAB method.
[Figure 11] Figure 11(a) is a diagram illustrating the relationship between the HER2 gene copy number calculated by the FISH method and a therapeutic effect (TTP [time to progression: time period when tumor growth is controlled by agent administration]) attained by trastuzumab. Figure 11(b) is a diagram illustrating the relationship between the amount of HER2 protein calculated by the IHC-QDs method and the therapeutic effect (TTP [time to progression: time period when tumor growth is controlled by agent administration]) attained by trastuzumab.

### Mode of Carrying Out the Invention

A method for determining effectiveness of a medicine containing an antibody as a component according to the present invention is not particularly limited as long as it is a method (excluding diagnostic activity of a doctor) employing a tissue staining method comprising the steps of: (a) labeling an antibody in the medicine containing an antibody as a component with a fluorescent material and contacting the fluorescence-labeled antibody with a tissue sample; (b) obtaining a fluorescence image by irradiating, with excitation light, a tissue site contacted with the antibody; (c) obtaining an autofluorescence image in the same field of view and at the same focus as in the fluorescence image in a close region on a shorter wavelength side or a longer wavelength side of an acquisition wavelength region of fluorescence emitted by the fluorescent material; and (d) obtaining a corrected fluorescence image by performing image processing for removing fluorescence brightness of the autofluorescence image from fluorescence brightness of the fluorescence image. Preferably, the method further comprises the steps of: (e) counting a number of cells in the tissue site contacted with the antibody; (f) identifying a single fluorescent particle on the basis of the fluorescence image and measuring average fluorescence brightness per fluorescent particle within the corrected fluorescence image corresponding to the single fluorescent particle; and (g) obtaining a number of fluorescent particles by dividing total fluorescence brightness within the corrected fluorescence image by the average fluorescence brightness per fluorescent particle, and calculating a number of fluorescent particles per cell by dividing the number of fluorescent particles by the number of cells counted in step (e). Examples of a subject to be given the medicine containing an antibody as a component include cancer patients, infectious disease patients and autoimmune disease patients, among which cancer patients are more suitable.

The antibody of the medicine containing an antibody as a component to be administered to a cancer patient is, for example, an antibody including, as an active ingredient, an antibody that recognizes a growth regulatory factor, a metastasis regulatory factor or the like of cancer as a target antigen and binds to the target antigen so as to suppress the growth of cancer cells or to kill cancer cells by the antibody binding to the cancer cells. An example of the medicine containing an antibody as a component includes, in addition to a medicine containing the aforementioned antibody as an active ingredient, a medicine containing an anticancer drug, an antiviral drug, an antibiotic or the like as an active ingredient and containing an antibody as delivery means to cancer cells. Preferable examples of the antibody medicine include Synagis, Remicade, Rituxan, trastuzumab, among which trastuzumab is more suitable. Examples of the cancer include colon cancer, rectal cancer, renal cancer, breast cancer, prostatic cancer, uterine cancer, ovarian cancer, endometrial cancer, esophageal cancer, hematological cancer, hepatic cancer, pancreatic cancer, skin cancer, lung cancer and breast cancer. Furthermore, as for the growth regulatory factor and the metastasis regulatory factor of cancer, examples of the growth regulatory factor of cancer include factors for regulating cell growth such as epidermal growth factors (EGF), EGF receptors (EGFR), platelet-derived growth factors (PDGF), PDGF receptors (PDGFR), insulin-like growth factors (IGF), IGF receptors (IGFR), fibroblast growth factors (FGF), FGF receptors (FGFR), vascular endothelial growth factors (VEGF), VEGF receptors (VEGFR), hepatocyte growth factors (HGF), HGF receptors (HGFR), neurotrophin (NT), transforming growth factor-β (TGFβ) family, human epidermal growth factor receptor 1, 2, 3 or 4 (HER1, 2, 3 or 4), macrophage colony-stimulating factor 1 (CSF1) and CSF1 receptors (CSF1R); and factors for regulating cell cycle such as cyclin, cyclin-dependent kinase (CDK), cyclin A, cyclin B, cyclin D, cyclin E, CDK1, CDK2, CDK4, CDK6, p16INK, p15, p21, p27 and RB (retinoblastoma). Examples of the metastasis regulatory factor of cancer include matrix metalloprotease 1 (MMP1), matrix metalloprotease 2 (MMP2), PAR1 (protease activated receptor 1), CXCR4 (chemokine [C-X-C motif] receptor 4) and CCR7 (chemokine [C-C motif] receptor 7). Among these, HER2 is suitable because trastuzumab targeting HER2 is widely used.

In order to determine effectiveness of a medicine containing an antibody as a component, for example, a gene diagnosis method is preferably employed together, and an obtained result may be examined by comparing with a result of a conventional determination method such as an immunohistochemistry method utilizing a coloring reaction caused between an enzyme and a substrate. Examples of the gene diagnosis method include PCR, southern hybridization and the FISH method. Examples of the immunohistochemistry method utilizing a coloring reaction caused between an enzyme and a substrate include methods utilizing horseradish peroxidase (HRP) and DAB (3,3'-diaminobenzidine), which is a substrate of HRP, alkaline phosphatase (ALP) and BCIP/INBT (5-bromo-4-chloro-3-indolyl phosphate/nitro blue tetrazolium), which is a substrate of ALP, and β-galactosidase and X-gal (5-bromo-4-chloro-3-indolyl β-galactopyranoside), which is a substrate of β-galactosidase. Among these, for determining effectiveness of trastuzumab against breast cancer, the FISH method and/or the immunohistochemistry method using HRP and DAB (the IHC-DAB method) are suitable for employing together.

In the method for determining effectiveness of a medicine containing an antibody as a component, the medicine to be determined for effectiveness is not limited to a medicine containing an antibody as an active ingredient, but includes a medicine containing an anticancer drug, an antiviral drug, an antibiotic or the like as an active ingredient and containing, as delivery means to cancer cells, an antibody recognizing protein specific to cancer cells, and a medicine containing an antibody targeting a factor of a signal transduction pathway related to a factor (protein) targeted by its active ingredient. If the antibody of the medicine containing an antibody as a component is HER2, examples of a factor present downstream of a signal transduction pathway of HER2 include the factors Ras and Raf related to cell growth and the anti-apoptotic factors PI3K and AKT.

As the antibody in the medicine containing an antibody as a component used in step (a) described above, an antibody specifically recognizing a growth regulatory factor or a metastasis regulatory factor of cancer, protein specific to cancer cells or the like is preferably used, and the antibody may be a monoclonal antibody or a polyclonal antibody. Furthermore, the class and the subclass of the antibody are not particularly limited, and examples of the class include IgA, IgG, IgE, IgD and IgM, and examples of the subclass include IgG1, IgG2, IgG3, IgG4, IgA1 and IgA2. Moreover, the term "antibody" herein has a meaning including any antibody fragments or derivatives, and includes, for example, Fab, Fab'₂, CDR, a humanized antibody, a multifunctional antibody and a single-chain antibody (ScFv). Such antibodies can be produced by known methods (see, for example, Harlow E. & Lane D., Antibody, Cold Spring Harbor Laboratory Press (1988)).

The fluorescent material used in step (a) described above is not particularly limited as long as it is a material that emits fluorescence under irradiation with radiation of UV, visible light or the like of a selected wavelength, and examples of an organic fluorescent material include fluorescein, rhodamine, Cy-5, Cy-3 and Alexa, and examples of an inorganic fluorescent material include fluorescent particles such as fluorescent silica nanoparticles and quantum dot fluorescent particles. Since the influence of autofluorescence is smaller when the wavelength is longer, a fluorescent material emitting fluorescence of a region close to a near infrared region and particularly of a near infrared region is preferably used, and an acquisition wavelength region of fluorescence emitted by the fluorescent material is preferably a wavelength region close to a near infrared region (570 to 800 nm) and particularly preferably a near infrared region (700 to 800 nm). Since the aforementioned organic fluorescent materials are unsuitable for analysis accompanied with long-term irradiation with excitation light because their fluorescence is rapidly faded by excitation light, it is preferable to use fluorescent particles, particularly quantum dot fluorescent particles, good in light stability and stable through long-term irradiation with excitation light. Any fluorescent particles may be used as long as they emit fluorescence under irradiation with excitation light, and the material and the like of the fluorescent particles are not limited. For example, particles made of a material itself emitting fluorescence, particles including a fluorescent material or particles coated with a fluorescent material for emitting fluorescence may be used. Furthermore, the shape and the size of the fluorescent particles are not particularly limited, and examples of the shape include a square shape, a disc shape, a polygonal shape and a spherical shape, among which a spherical shape is preferred. As for the size, the diameter of a spherical particle is 0.00001 nm to 1 mm, preferably 0.001 nm to 100 µm and more preferably 0.01 nm to 10 µm. The wavelength of the fluorescence emitted by the fluorescent particles may be appropriately selected. The fluorescent particles may be produced by a method described in Japanese unexamined Patent Application Publication No. 9-241634 or 2010-242059. Alternatively, a commercially available product such as SPHERO fluorescent particles (manufactured by Bay bioscience Inc.) prepared by staining polystyrene particles with an appropriate fluorescent dye in the presence of polystyrene core particles or by polymerizing styrene with a fluorescent dye, or fluorescent particles Estapor (registered trademark) standard microspheres (manufactured by Merck Chime SAS) may be obtained. Among various fluorescent particles, quantum dot fluorescent particles also designated as colloidal quantum dot (QD) are preferably used because such fluorescent particles have both extremely sharp emission spectra and high quantum efficiency. The quantum dot fluorescent particles are photogenic semiconductor nanoparticles having a diameter ranging from 1 to 20 nm, and are utilized for fluorescence imaging in the field of biology and medical diagnosis. In the quantum dot fluorescent particles, electrons are quantally confined in three-dimensional and nano-sized semiconductor crystal with a clear profile, and as the size of the quantum dot fluorescent particles is smaller, the band gap of the semiconductor is larger, and therefore, the photoluminescence emission wavelength of the semiconductor can be adjusted in accordance with the size over the whole visible spectra. The quantum dot fluorescent particles of the present invention may be any photogenic semiconductor nanoparticles, and the particle size is preferably appropriately adjusted in accordance with the wavelengths of the excitation light and the fluorescence. As the quantum dot fluorescent particles, for example, Qdot (registered trademark) 525, Qdot 565, Qdot 585, Qdot 605, Qdot 625, Qdot 655, Qdot 705 or Qdot 800 (all manufactured by Invitrogen Corporation), that is, nanocrystal of a semiconductor material (CdSe) covered by a semiconductor shell (ZnS), may be used. If the fluorescence wavelength to be obtained exceeds 800 nm, camera sensitivity is degraded, and therefore, among quantum dot fluorescent particles emitting fluorescence of the near infrared region, quantum dot fluorescent particles emitting fluorescence of 705 nm (Qdot 705) are preferably used.

Examples of a method for labeling the antibody with a fluorescent material in step (a) described above include a method using an antibody (a secondary antibody) having affinity with the antibody, a biotin-avidin method, a thiol-maleimide coupling reaction method, a method using an existing chemical linker, a crosslinking reaction method using a crosslinking agent (such as EDC) and an ionic bonding method. If the antibody is a humanized antibody or a human antibody, the thiol-maleimide coupling reaction method is preferably employed among these.

The tissue sample used in step (a) described above may be, for example, a tissue sample fixed section such as a formalin fixed paraffin section or a frozen section, and specific examples include a pathological tissue sample, a peripheral blood tissue sample, a cell block sample of cells separated from a tissue or cells separated from a body fluid, a cell block sample prepared by precipitating or centrifuging a suspension sample of peripheral blood leukocyte or cells, a sealed sample of a tissue, and a smear sample of a liquid including peripheral blood, a body fluid, exudate or cells.

A method for contacting the fluorescence-labeled antibody with the tissue sample in step (a) described above is not particularly limited, and since an antigen-antibody reaction is caused through this contact, the temperature at the time of contact is preferably 0 to 40°C. The contact time depends upon the type of tissue sample to be used, the concentration of the antibody, the height of a titer, the contact (reaction) temperature, the amount of a target factor (antigen), location and the like, and is not particularly limited but may be appropriately selected, and is, for example, 10 minutes to 12 hours and preferably 10 minutes to 2 hours. Furthermore, in order to prevent non-specific binding of the antibody, a blocking treatment with FBS, BSA, IgG or the like may be conducted before contacting the antibody with the tissue sample.

An excitation wavelength of the excitation light employed in step (b) described above is shorter than the fluorescence wavelength emitted by the fluorescent material, and from the viewpoint of safety for a human body, is preferably 400 to 700 nm and more preferably 450 to 650 nm. Specifically, a generally employed excitation wavelength of 488 nm, 532 nm or 635 nm may be employed.

Examples of the fluorescence image used in step (b) described above include a fluorescence still image and a fluorescence dynamic image corresponding to the fluorescence still image. The fluorescence still image can be obtained by using a fluorescence microscope capable of, for example, controlling imaging conditions for a CCD camera, forming and displaying a captured fluorescence still image and controlling light quantity of a light source with a computer such as a general PC used as a controller. Necessary setting for a filter disposed on the side of the excitation light, a dichroic mirror and the like is conducted so as not to saturate the fluorescence brightness of the fluorescent material, and furthermore, the exposure time of the excitation light is set preferably to be ranging between 0.03 and 30 to 60 seconds. As a method for confirming whether or not the fluorescence brightness of the fluorescent material in each pixel is saturated, a precedently specified brightness value or a brightness value corresponding to a precedently specified ratio to the maximum brightness value (for example, in case of an 8-bit image, the maximum value is 255 and hence, a value of 220 or a ratio of 90% of the maximum brightness value) is set, so that it may be determined that the brightness is saturated when the brightness exceeds the set value. Besides, the determination may be made in this case not by using merely one pixel but by using a plurality of pixels corresponding to a precedently specified ratio in the whole image. For identifying whether or not the fluorescence brightness of the fluorescent material in a fluorescence still image derives from one particle, a fluorescence dynamic image may be obtained if necessary. As the fluorescence dynamic image, it is preferable to capture 50 to 200 frames of a dynamic image in the same field of view and at the same focus as in the fluorescence still image with resolution of 2 to 500 msec. As the band-pass filter used for obtaining the fluorescence image, a band-pass filter for selectively passing fluorescence of a specific wavelength of red, cyan, orange, green, blue or the like emitted by the fluorescent material is used. It is preferable to use a band-pass filter capable of obtaining 25% or more, preferably 50% or more and more preferably 75% or more of the total fluorescence brightness of the fluorescent material. More preferably, as the band-pass filter used for obtaining the fluorescence image, a band-pass filter capable of obtaining 30% of the fluorescence brightness of the fluorescent material in a wavelength region of 30 nm or smaller, preferably 15 nm or smaller and more preferably 5 nm or smaller is used. If a fluorescent material emitting fluorescence of, for example, 705 nm is used, the band-pass filter for obtaining the fluorescence image is preferably a band-pass filter having a pass range of a fluorescence wavelength of 695 to 740 nm.

As a method for obtaining the autofluorescence image in step (c) described above, the band-pass filter used for obtaining fluorescence wavelengths emitted by the fluorescent material is exchanged with a band-pass filter capable of obtaining wavelengths of a close region on a shorter or longer wavelength side, preferably a close region on a shorter wavelength side, as compared with the band-pass filter used for obtaining the fluorescence image, and an image is obtained in the same field of view at the same focus as in the fluorescence still image. Thus, an image not including the fluorescence of the fluorescent material but including autofluorescence alone is obtained. Fluorescence of longer wavelengths is less refracted than fluorescence of shorter wavelengths and is focused on a farther point than the fluorescence of shorter wavelengths, and therefore, if the acquisition wavelength region of fluorescence emitted by the fluorescent material is largely different, in wavelength, from the wavelength region used for obtaining the autofluorescence image, the focus of the autofluorescence included in the fluorescence still image is largely shifted from the focus of the autofluorescence included in the autofluorescence image. Therefore, a difference in the wavelength between the acquisition wavelength region of fluorescence emitted by the fluorescent material and the close region is suitably 130 nm or smaller, preferably 120 nm or smaller, more preferably 110 nm or smaller, and particularly preferably 100 nm or smaller. Examples of the band-pass filters used for obtaining the fluorescence image and the autofluorescence image of the present invention include band-pass filters having pass ranges of fluorescence wavelengths of specifically 505 to 545 nm, 565 to 595 nm, 585 to 630 nm, 640 to 690 nm, 695 to 740 nm and 760 to 800 nm. If the fluorescence still image is obtained by using, for example, a band-pass filter having a pass range of a fluorescence wavelength of 695 to 740 nm, a band-pass filter having a pass range of a fluorescence wavelength of 640 to 690 nm is preferably used for obtaining the autofluorescence image. The image obtaining conditions for obtaining the autofluorescence image are set so that, when the brightness of the autofluorescence is compared in the corresponding areas between the fluorescence still image and the autofluorescence image, the maximum brightness of the autofluorescence in an arbitrary ROI, for example, an area of 25 × 25 pixels, can be higher in the autofluorescence image than in the fluorescence still image by 1.1 to 1.3 times and preferably 1.2 times. Specifically, the brightness of the autofluorescence is higher in the autofluorescence image than in the fluorescence still image preferably by 10 to 30% and particularly approximately 20%.

As a method for obtaining the corrected fluorescence image in step (d) described above, the fluorescence still image and the autofluorescence image are converted into image files of JPEG, TIF or the like, and then, on the basis of the brightness of the autofluorescence image, the brightness of the autofluorescence is subtracted from the brightness of the fluorescence still image by using image analysis software such as Image J (http://rsb.info.nih.gov/ij/), Photoshop (manufactured by Adobe Systems Incorporated.), After Effect (manufactured by Adobe Systems Incorporated.) or G-Count (manufactured by G-Angstrom). Incidentally, in converting the image into an image file, a threshold value is selected preferably so that the whole fluorescence distribution may be covered.

As a method for counting the number of cells in the tissue site contacted with the antibody in step (e) described above, for example, after staining cell nuclei with a nucleic acid stain bound to DNA for emitting fluorescence, such as DAPI, Hoechst or PI (Propidium Iodide), the total number of stained cell nuclei observed in the same field of view as in the fluorescence image is counted. If the acquisition wavelength region of fluorescence emitted by the fluorescent material is the near infrared region, DAPI or Hoechst that emits blue fluorescence is more preferably used than PI that emits red fluorescence having a similar fluorescence characteristic to the fluorescence emitted by the fluorescent material.

Examples of a method for identifying a single fluorescent particle in step (f) described above include a method in which the fluorescence brightness of the fluorescent particles in the fluorescence image is measured so as to determine, on the basis of brightness information of fluorescence emitted by a single fluorescent particle, how many particles the fluorescent particles correspond to, or a method utilizing a "blinking property" of fluorescent particles. If the fluorescent particle is a quantum dot fluorescent particle, the method utilizing the blinking property is preferably employed. The "blinking property" herein refers to a property in which OFF time (extinct time) when the emission intensity against the irradiation time or the irradiation amount in the excitation light irradiation becomes suddenly substantially zero (OFF) is continuously in this state for approximately several msec. to 5 sec. and then, the original emission intensity is attained again, so that such OFF time and such ON time may be alternately repeated. Furthermore, the method for identifying a single fluorescent particle by utilizing the "blinking property" means a method in which the extinct time of fluorescent particles attained under irradiation with excitation light of arbitrary time of, for example, 10 to 100 seconds is measured on the basis of a fluorescence still image or a fluorescence dynamic image, so as to evaluate whether or not the fluorescent particles correspond to a single particle, and thus, a single fluorescent particle is specified. For example, if quantum dot fluorescent particles emitting fluorescence of 705 nm are used as the fluorescent particles, the extinct time of a single quantum dot fluorescent particle attained under irradiation with excitation light for 20 seconds is approximately 4 seconds, and therefore, a fluorescent particle having extinct time of approximately 4 seconds can be specified as a quantum dot fluorescent particle derived from a single particle. Here, in order to determine whether or not a quantum dot fluorescent particle has extinguished, a threshold value of the fluorescence brightness of the quantum dot fluorescent particles may be appropriately set. For example, 0 to 30%, preferably 5 to 15% and more preferably 10% of the maximum fluorescence brightness of the quantum dot fluorescent particles is set as the threshold value. The fluorescence brightness corresponding to the specified single particle in the corrected fluorescence image is measured by using image analysis software such as Image J, Photoshop, After Effect or G-Count. The calculation of average fluorescence brightness per particle through this measurement is performed by using 2 to 200 fluorescent particles and preferably 5 to 10 fluorescent particles in consideration of labor and accuracy.

In step (g) described above, the number of fluorescent particles per cell is calculated. The number of fluorescent particles per cell can be calculated by assigning a value of the total fluorescence brightness of the quantum dot fluorescent particles obtained in the corrected fluorescence image (the total fluorescence brightness in a screen), the number of cells obtained in the cell nucleus stained image and a value of average fluorescence brightness per quantum dot fluorescent particle (single particle fluorescence brightness) in an expression [(the total fluorescence brightness in a screen/single particle fluorescence brightness)/the number of cells = the total number of fluorescent particles in the image/the number of cells = the number of fluorescent particles/cell].

In accordance with the determination method of the present invention comprising the steps (a) to (d), corrected fluorescence images of a tissue sample of a determination object and a normal tissue sample are obtained so as to compare fluorescence brightness between the corrected fluorescence images. If the fluorescence brightness of the corrected fluorescence image of the tissue sample of the determination object is higher, it suggests that there is a large amount of protein recognized by the antibody in the medicine, and therefore, it is determined that the medicine can be expected to attain a therapeutic effect in the determination object or that the determination object is suitable as a patient eligible for administration of the medicine. Furthermore, according to the determination method of the present invention comprising the steps (a) to (g), the numbers of fluorescent particles per cell in a tissue sample of a determination object and a normal tissue sample are calculated so as to compare the numbers with each other. If the number of fluorescent particles per cell in the tissue sample of the determination object is larger, it suggests that there is a large amount of protein recognized by the antibody in the medicine, and therefore, it is determined that the medicine can be expected to attain a therapeutic effect in the determination object or that the determination object is suitable as a patient eligible for administration of the medicine. For example, an anti-HER2 antibody used as an active ingredient of trastuzumab, that is, an antibody medicine for breast cancer, is labeled with quantum dot fluorescent particles, and the antibody labeled with the quantum dot fluorescent particles is used for obtaining the number of fluorescent particles per cell detected by trastuzumab. The obtained number can be used as an index for selecting a patient eligible for administration of trastuzumab or for estimating a therapeutic effect attained by trastuzumab.

Examples of the excitation light irradiation means used in the determination system include a mercury lamp (100 V), a mercury lamp (200 V), a xenon lamp (75 V), a xenon lamp (150 V), a halogen lamp (12 V-100 W), a tungsten lamp (6 V-30 W), a xenon lamp [having a wavelength of 250 to 1,000 nm], a tungsten lamp [having a wavelength of 250 to 1,000 nm], a Cr:LiSAF lamp [having a wavelength of 430 nm], a helium-cadmium laser [having a wavelength of 325 or 442 nm], a UV argon laser [having a wavelength of 351 or 364 nm], an argon ion laser [having a wavelength of 488 or 514 nm], a helium neon laser [having a wavelength of 543, 594 or 633 nm], a krypton ion laser [having a wavelength of 568 or 647 nm] and an LD-pumped CW/Q-CW (continuous wave oscillation/quasi-continuous wave oscillation) solid-state laser [having a wavelength of 375 nm, 405, 440, 473, 488, 505, 515, 532, 561, 594, 635, 785 or 1064 nm]. Among these, an excitation wavelength of 400 to 700 nm is preferred from the viewpoint of safety to a human body, and preferably, an LD-pumped CW/Q-CW solid-state laser [having a wavelength of 488, 532 or 635 nm] is suitably used.

Furthermore, examples of the fluorescence image obtaining means used in the determination system include a fluorescence microscope and a confocal laser scanning fluorescence microscope. Examples of a fluorescence image obtained by such fluorescence image obtaining means include a fluorescence still image and an autofluorescence image, and a fluorescence dynamic image if necessary.

Furthermore, the band-pass filter for obtaining a fluorescence image used in the determination system is, for example, a band-pass filter for obtaining the fluorescence still image (the fluorescence dynamic image) or the autofluorescence image. Besides, the band-pass filter for obtaining a cell nucleus fluorescence image is varied depending upon the fluorescence characteristics of a nucleic acid stain to be used. In considering that, for example, the maximum fluorescence wavelength of DAPI is 461 nm, the maximum fluorescence wavelength of Hoechst 33342 and Hoechst 33258 is 465 nm and the maximum fluorescence wavelength of PI (propidium iodide) is 617 nm, a band-pass filter capable of sufficiently covering such a wavelength is preferably used.

The present invention will now be specifically described with reference to examples, but it is noted that the technical scope of the present invention is not limited to the following examples.

### Examples

### Example 1

### [Method for preparing tissue sample immunostained by IHC-QDs method]

As human breast cancer pathological tissues, 6 cases evaluated based on the IHC-DAB method as Score 0, 6 cases evaluated as Score 1, 11 cases evaluated as Score 2 and 14 cases evaluated as Score 3, namely, 37 cases in total, were selected. These cases were selected carefully so that the background of the cases might be well-balanced as shown in Table 1. Tissue samples of tissues of the 37 cases were prepared by a method generally employed for pathological tissue diagnosis. Specifically, a breast tissue specimen of a cancer patient was fixed with formalin, followed by dehydration with alcohol and a xylene treatment, and the resultant was immersed in paraffin at a high temperature for paraffin embedding, and thus, a tissue sample was prepared (Figure 1(a)). Subsequently, the tissue sample was cut into sections of 2 to 4 µm, followed by deparaffinization with xylene and an alcohol treatment, and the resultant was washed with deionized water. In order that an antibody could make an efficient access to an antigenic site in the fixed tissue, the sample was retrieved in a 10 mM citrate solution (pH 6.0) at 121°C for 15 minutes for relaxing the structure of the fixed tissue. Subsequently, the sample was washed successively with deionized water and phosphate buffered saline (PBS), followed by an antibody reaction conducted at 25°C for 3 hours in a PBS solution including 15 nM of a HER2 antibody, which is an active ingredient of trastuzumab, labeled with quantum dot fluorescent particles (Qdot 705) emitting fluorescence of 705 nm (trastuzumab + Qdot 705). Here, as a method for labeling trastuzumab with the Qdot 705, a coupling reaction between a thiol group of reduced trastuzumab and a maleimide group of the quantum dot fluorescent particles was employed (Figure 1(b)). Incidentally, human IgG labeled with the Qdot 705 (human IgG + Qdot 705) was used as a control. After washing with a PBS solution four times, cell nuclei were stained by DAPI staining (2 µg/ml PBS) at 25°C for 10 minutes for counting the number of cells. After washing with PBS three times, the resultant was mounted, and thus, a tissue sample stained by the immunostaining method (IHC-QDs method) was prepared (Figure 1(a)).

**[Table 1]**

| Characteristics | |
|---|---|
| Age | |
| Median (range) | 55 (25-88) y |
| Stage | |
| I | 13 (35%) |
| II (IIA + IIB) | 13 (35%) |
| III (IIIA + IIIB + IIIC) | 9 (24%) |
| IV | 2 (6%) |
| ER/PgR status | |
| ER+ and/or PgR+ | 18 (49%) |
| ER- and PgR- | 19 (51%) |
| HER2 status by IHC-DAB‡ | |
| Score 0 | 6 (16%) |
| Score 1 | 6 (16%) |
| Score 2 | 11 (30%) |
| Score 3 | 14 (38%) |

The aforementioned "stage" corresponding to breast cancer staging is determined on the basis of invasiveness of cancer (depth of cancer) and metastasis state, and is classified into eight stages of 0, I, IIA, IIB, IIIA, IIIB, IIIC and IV in accordance with a combination of three elements (1. primary focus (size): factor T, 2. presence of metastasis to lymph node: factor N, and 3. presence of other metastasis (distant metastasis): factor M).

### Example 2

### [Problem of immunofluorescence tissue staining method]

Each of the immunostained tissue samples prepared in Example 1 was irradiated with excitation light of 488 nm by using an apparatus obtained by combining a confocal unit (manufactured by Yokogawa Electric Corporation), a fluorescence microscope (manufactured by Olympus Corporation) and an electron-multiplier CCD (EM-CCD) camera (manufactured by Andor Technology), and thereafter, a fluorescence image (a fluorescence still image) of the quantum dot fluorescent particles (used for labeling trastuzumab) was obtained by using a band-pass filter having a pass range of a wavelength of 695 to 740 nm. Figure 2 exemplarily illustrates a case evaluated as Score 3 by the IHC-DAB method, and thus, fluorescence derived from the quantum dot fluorescent particles was observed if trastuzumab + Qdot 705 was used while fluorescence derived from the quantum dot fluorescent particles was minimally observed if the control of human IgG + Qdot 705 was used. Such a result reveals that trastuzumab specifically binds to HER2 protein and shows that trastuzumab + Qdot 705 can specifically detect HER2 protein expressed in breast cancer cells. On the other hand, since there was background of autofluorescence in addition to the fluorescence derived from the quantum dot fluorescent particles, there arose necessity for excluding the autofluorescence. The autofluorescence is largely varied depending upon the type of tissue sample and the position in the tissue sample, and hence, a constant threshold value cannot be set for the autofluorescence. Accordingly, in order to accurately measure fluorescence brightness of the quantum dot fluorescent particles (used for labeling trastuzumab), it is necessary to exclude the autofluorescence.

As a method for removing influence of autofluorescence in the biological tissue staining, for example, (a) an autofluorescence fading method through excitation light irradiation and (b) a contrast adjusting method are known (Figure 3). In (a) the autofluorescence fading method through excitation light irradiation, owing to the light stability of the quantum dot fluorescent particles, the autofluorescence alone may be faded without degrading the brightness derived from the quantum dot fluorescent particles even through long-term light irradiation. It takes, however, a long time to completely fade the autofluorescence, and in addition, this method has a problem in which the autofluorescence may be faded on an irradiated face alone. In (b) the contrast adjusting method, if the fluorescence brightness of the quantum dot fluorescent particles is sufficiently higher than the brightness of the autofluorescence, it is possible to extinguish the brightness of the autofluorescence alone. If, however, there is no sufficient difference between the brightness of the quantum dot fluorescent particles and the brightness of the autofluorescence, the fluorescence brightness of the quantum dot fluorescent particles may be extinguished by adjusting contrast. Furthermore, the autofluorescence is largely varied depending upon the type of tissue sample and the position in the tissue sample, and hence, a constant threshold value cannot be set for the autofluorescence.

### Example 3

### [Generation of corrected fluorescence image excluding autofluorescence]

It is a fluorescence still image in which the autofluorescence of the background has fluorescent intensity of zero (0) that is necessary. If such an image is obtained, the total fluorescence included in the fluorescence still image can be calculated as the fluorescence derived from the quantum dot fluorescent particles. The contrast adjusting method described in Example 2 is conducted as division of the fluorescent intensity, and zero (0) cannot be obtained by division, and hence, an image processing method using subtraction, which can give zero (0), is necessary. Therefore, as an image processing method for removing autofluorescence from a fluorescent still image, the following method was devised: First, a breast cancer tissue sample immunostained with quantum dot fluorescent particles was irradiated with excitation light (laser) of an excitation wavelength of 488 nm, so as to obtain a fluorescence image of the quantum dot fluorescent particles (fluorescence of the quantum dot fluorescent particles + autofluorescence) by using a band-pass filter having a pass range of a fluorescence wavelength of 695 to 740 nm. Thereafter, an autofluorescence image including autofluorescence alone was obtained on exactly the same focal surface and in the same field of view as in the fluorescence image by using a band-pass filter having a pass range of a fluorescence wavelength of 640 to 690 nm. Subsequently, the fluorescence image (the fluorescence of the quantum dot fluorescent particles + the autofluorescence) and the autofluorescence image thus obtained were fetched as images each of 512 × 512 pixels and converted into JPEG images of 256 gray levels (Figure 4(a)). Thereafter, brightness information (a value of 0 to 255) included in each pixel of the autofluorescence image (the autofluorescence) was subtracted from brightness information (a value of 0 to 255) included in a corresponding pixel of the fluorescence still image (the fluorescence of the quantum dot fluorescent particles + the autofluorescence), so as to generate an image including the fluorescence of the quantum dot fluorescent particles alone (a corrected fluorescence image).

In order to generate such a corrected fluorescence image, the fluorescence wavelength characteristics of the autofluorescence in various wavelength ranges were first examined. After cancer tissue samples not immunostained with quantum dot fluorescent particles were excited with an excitation wavelength of 488 nm, autofluorescence images of the same field of view and the same focus were obtained by using six kinds of band-pass filters having pass ranges of fluorescence wavelengths respectively of 505 to 545 nm, 565 to 595 nm, 585 to 630 nm, 640 to 690 nm, 695 to 740 nm and 760 to 800 nm. With respect to all the autofluorescence images obtained by using the six kinds of band-pass filters, images in the same region of 512 × 512 pixels were converted into JPEG images, and from each of the JPEG images, an image of the same region of 512 × 420 pixels (210540 pixels in total) was cut out, an average value of fluorescence brightness in this image was obtained so as to calculate a value of "average fluorescence brightness of the autofluorescence image/pixel" (value 1)). Furthermore, with respect to each of the autofluorescence images obtained by using the six kinds of band-pass filters, an image of background excluding the autofluorescence was obtained as a background fluorescence image, and fluorescence brightness of the background fluorescence image was measured. In this measurement, fluorescence brightness in a region of 25 × 25 pixels of the background fluorescence image was obtained in arbitrary three regions, so as to calculate a value of "average fluorescence brightness of the background fluorescence image/pixel" (value 2)). Such three regions were set to be corresponding regions in all the autofluorescence images obtained by using the six kinds of band-pass filters. These values 1) and 2) were used for calculating average fluorescence brightness of the autofluorescence excluding the fluorescence brightness of the background fluorescence (hereinafter simply referred to as the "fluorescence brightness of the autofluorescence") on the basis of an expression "(average fluorescence brightness of the autofluorescence image/pixel - average fluorescence brightness of the background fluorescence image/pixel) × the number of pixels" = "(value 1) - value 2)) × the number of pixels". The fluorescence brightness of the autofluorescence obtained by using each of the six kinds of band-pass filters was calculated, and the calculated fluorescence brightness was divided by the wavelength width of the pass range of the fluorescence wavelength of the corresponding band-pass filter (namely, 505-545 nm: 40 nm, 565-595 nm: 30 nm, 585-630 nm: 45 nm, 640-690 nm: 50 nm, 695-740 nm: 45 nm, or 760-800 nm: 40 nm), so as to calculate the average fluorescence brightness of the autofluorescence per wavelength width. The fluorescence brightness of the autofluorescence per wavelength width thus obtained were plotted at the centers of the pass ranges of the fluorescence wavelengths of the respective band-pass filters, and the plotted points were connected with a dotted line. Thus, a diagram illustrating the fluorescence wavelength characteristics of the autofluorescence was drawn (Figure 4(b)).

Next, in order to make the fluorescence wavelength characteristics of the quantum dot fluorescent particles correspond to the fluorescence wavelength characteristics of the autofluorescence, a breast cancer tissue sample immunostained with quantum dot fluorescent particles in the same manner as in Example 1 was excited with excitation light of 488 nm, and a fluorescence still image (the quantum dot fluorescent particles + autofluorescence) was obtained by using a band-pass filter having a pass range of a fluorescence wavelength of 695 to 740 nm. Subsequently, a fluorescence dynamic image of the same field of view and the same focus as in the fluorescence still image (the quantum dot fluorescent particles + the autofluorescence) was obtained, and this fluorescence dynamic image was used for identifying a single particle of the quantum dot fluorescent particles in the same manner as in Example 4 described below (namely, by a method using the blinking reaction of the quantum dot fluorescent particles). Thereafter, the fluorescence still image was used for measuring fluorescence brightness of the specified single particle of the quantum dot fluorescent particles. Specifically, attention is paid to a single particle of the quantum dot fluorescent particles not overlapping autofluorescence, and fluorescence brightness of a pixel region (of 9 to 25 pixels) covering the whole fluorescence of the single particle of interest was obtained, so as to calculate a value of "fluorescence brightness of the fluorescence still image/pixel) (value 3)). Furthermore, in the fluorescence still image, an image of background including neither the autofluorescence nor the fluorescence of the quantum dot fluorescent particles was obtained as a background fluorescence image, and fluorescence brightness of the background fluorescence image was measured. In this measurement, fluorescence brightness was obtained in arbitrary three regions each having the same number of pixels as that employed in measuring the fluorescence brightness of the single particle of the quantum dot fluorescent particles, so as to calculate a value of "average fluorescence brightness of the background fluorescence image/pixel" (value 4)). The values 3) and 4) were used for calculating fluorescence brightness of the quantum dot fluorescent particles excluding the fluorescence brightness of the background fluorescence (hereinafter simply referred to as the "fluorescence brightness of the quantum dot fluorescent particles") on the basis of an expression "(average fluorescence brightness of the fluorescence still image/pixel - average fluorescence brightness of the background fluorescence image/pixel) × the number of pixels" = "(value 3) - value 4)) × the number of pixels". Moreover, in a region not including the fluorescence of the quantum dot fluorescent particles but including the autofluorescence alone in the fluorescence still image (the quantum dot fluorescent particles + the autofluorescence), fluorescence brightness in arbitrary three regions each having the same number of pixels as that employed in measuring the fluorescence brightness of the single particle of the quantum dot fluorescent particles was obtained, so as to calculate a value of "average fluorescence brightness of the autofluorescence image/pixel" (value 5)). The value 5) was used for calculating fluorescence brightness of the quantum dot fluorescent particles against the fluorescence brightness of the autofluorescence on the basis of an expression "fluorescence brightness of the quantum dot fluorescent particles/([average fluorescence brightness of the autofluorescence image/pixel] × the number of pixels)" = ([value 3) - value 4)] × the number of pixels)/([value 5) - value 4)] × the number of pixels). The processing described so far was conducted on fifteen different fluorescence still images (quantum dot fluorescent particles + autofluorescence), and on the basis of the thus obtained values of fifteen fluorescent particles, an average (i.e., a fluorescence brightness average of the quantum dot fluorescent particles against the fluorescence brightness of the autofluorescence) was obtained and calculated as 1.88 ± 0.10 (an average value ± s.e.m.). As for change in the fluorescence brightness of the quantum dot fluorescent particles (Qdot 705), for example, description given in a pamphlet (file name: 2008-20-CBQdot) held in a website of Invitrogen Corporation (http://www.invitrogen.jp/qdot/index.shtml) was referred to, and areas having the fluorescence brightness of the quantum dot fluorescent particles and the fluorescence brightness of the autofluorescence were determined so that a ratio of (an area having fluorescence brightness of Qdot 705)/(an area having fluorescence brightness of autofluorescence) can be 1.88. Thus, a diagram illustrating the fluorescence wavelength characteristics of the Qdot 705 together with those of the autofluorescence was drawn (Figure 4(b)). Incidentally, for determining these areas, an area having the fluorescence brightness of the autofluorescence of a fluorescence wavelength of 695 to 740 nm was assumed to be a rectangle for reasons of expediency. When the ratio of (an area having fluorescence brightness of Qdot 705)/(an area having fluorescence brightness of autofluorescence) is 1.88, a ratio occupied by the fluorescence brightness of the autofluorescence of the background exceeds 50% in the total fluorescence brightness of the fluorescence still image (the fluorescence brightness of the quantum dot fluorescent particles + the fluorescence brightness of the autofluorescence). In this manner, it was reconfirmed that it is necessary to remove the fluorescence brightness of the autofluorescence for quantitatively determining the fluorescence brightness of the quantum dot fluorescent particles.

A corrected fluorescence image excluding the autofluorescence was generated in accordance with the following procedure: A tissue sample obtained by the IHC-QDs method was irradiated with excitation light of 488 nm for 40 seconds, so as to obtain a fluorescence still image (quantum dot fluorescent particles + autofluorescence) (40 s/frame × one image) by using a band-pass filter having a pass range of a fluorescence wavelength of 695 to 740 nm and an autofluorescence image (40 s/frame × one image) of the same field of view and the same focus as in the fluorescence still image by using a band-pass filter having a pass range of a fluorescence wavelength of 640 to 690 nm. In order to count the number of cells, the tissue sample was excited with excitation light of 400 nm, so as to obtain a cell nucleus image (detected with DAPI) by using a band-pass filter having a pass range of a fluorescence wavelength of 420 to 500 nm (as illustrated as an image on the lower right side in Figure 5). The autofluorescence was compared in the same region between the fluorescence still image (the quantum dot fluorescent particles + the autofluorescence) and the autofluorescence image, so that the maximum value of the autofluorescence in an arbitrary ROI (such as an area of 25 × 25 pixels) could be "the fluorescence still image (the quantum dot fluorescent particles + the autofluorescence) × 1.2 = the autofluorescence image". Specifically, the brightness of the autofluorescence was set to be higher in the autofluorescence image by approximately 20% than in the fluorescence still image (the quantum dot fluorescent particles + the autofluorescence). In this manner, with the brightness derived from the autofluorescence adjusted, the images were converted into JPEG images (as illustrated as images on the upper left side and upper right side in Figure 5). Incidentally, in converting the images into the JPEG images, a threshold value was set so as to cover the whole fluorescence. In order to obtain a corrected fluorescence image excluding the autofluorescence and including the quantum dot fluorescent particles alone, processing of "the fluorescence still image (the quantum dot fluorescent particles + the autofluorescence) - the autofluorescence image" was conducted by using image analysis software of Photoshop (as illustrated as an image on the lower left side in Figure 5). At this point, it was confirmed, in arbitrary plural regions, that brightness corresponding to a single particle of the quantum dot fluorescent particles had not been extinguished and that the fluorescent intensity of an autofluorescence portion was zero (0) after the subtraction. In this manner, the fluorescence still images (the quantum dot fluorescent particles + the autofluorescence) of the tissue sample having been subjected to the pathological diagnosis by the IHC-DAB method and the corrected fluorescence images were obtained (Figure 6).

### Example 4

### [Method for calculating average fluorescence brightness per particle of quantum dot fluorescent particles]

In order to calculate average fluorescence brightness per particle of the quantum dot fluorescent particles, it is necessary to determine whether or not fluorescence brightness is derived from a single particle, and for this purpose, the "blinking property" peculiar to the quantum dot was herein utilized. If 10% of the maximum fluorescence brightness of the quantum dot fluorescent particles is set as a threshold value corresponding to a boundary of blinking (between light and darkness) (see an arrowhead of Figure 7), an off-state (a dark state) of a single particle of the quantum dot fluorescent particles corresponds to approximately 4 seconds with irradiation time of 20 seconds (Figure 7). Therefore, the fluorescence brightness per particle can be obtained by measuring average fluorescent intensity of particles each having an off-state (a dark state) of approximately 4 seconds. Accordingly, in order to obtain a fluorescence dynamic image corresponding to a fluorescence still image, after the irradiation with excitation light, 100 continuous fluorescence still images were obtained with resolution of 200 to 400 msec. by using a band-pass filter having a pass range of a fluorescence wavelength of 690 to 730 nm, so as to obtain a fluorescence dynamic image (200-400 ms/frame × 100 images). The thus obtained fluorescence dynamic image was used for identifying a particle having an off-state of approximately 4 seconds as a single fluorescent particle, and after the specification, fluorescence brightness of a corresponding particle in the still image was determined by using image analysis software Image J. This processing was conducted on 5 to 10 particles, so as to calculate average fluorescence brightness per particle.

### Example 5

### [Method for calculating the number of particles per cell]

The number of quantum dot fluorescent particles per cell can be calculated by assigning the value of the total fluorescence brightness of the quantum dot fluorescent particles obtained from a corrected fluorescence image (total fluorescence brightness within a screen), the number of cells measured based on a cell nucleus stained image (detected with DAPI) and the value of average fluorescence brightness per particle of the quantum dot fluorescent particles (single particle fluorescence brightness) in an expression [(the total fluorescence brightness within a screen/single particle fluorescence brightness)/the number of cells = the total number of the fluorescent particles within the image/the number of cells = the number of fluorescent particles/cell]. Furthermore, similar processing was performed by using human IgG labeled with the quantum dot fluorescent particles to be used as a control, so as to calculate "the number of quantum dot fluorescent particles used for labeling trastuzumab/cell - the number of quantum dot fluorescent particles used for labeling human IgG/cell". In this manner, the number of non-specific particles was removed from the number of quantum dot fluorescent particles (used for labeling trastuzumab), so as to calculate the true number of fluorescent particles per cell.

### Example 6

### [Comparison of conventional diagnosis method for breast cancer and IHC-QDs method]

In order to check whether or not the amount of HER2 protein recognized by the HER2 antibody, that is, an active ingredient of trastuzumab, calculated by the IHC-QDs method is in correlation with the HER2 gene copy number, the HER2 gene copy number was measured by the FISH method. The FISH method was conducted by the following procedure: the aforementioned tissue samples of the 37 cases having been subjected to the IHC-QDs method were deparaffinized by three treatments with Hemo-De at room temperature for 10 minutes, followed by two washings with 100% ethanol at room temperature for 5 minutes for dehydration. In order to improve the reachability of a DNA probe, each of the tissue samples was subjected successively to the following treatments 1) to 3): 1) Removal of protein of the cell membrane and the nuclear membrane (with 0.2 N HCl at room temperature for 20 minutes); 2) decomposition of a crosslinked structure with formalin (with 1 M NaSCN at 80°C for 30 minutes); and 3) decomposition of protein of the cell membrane and the nuclear membrane, particularly collagen (with 50 ml of 25 mg protease [2500-3000 units/mg][pepsin]/1 M NaCl [pH 2.0] at 37°C for 60 minutes). In order to convert the DNA into a single strand, each of the tissue samples was treated with a degeneration solution (70% formamide/SSC [150 mM NaCl, 15 mM sodium citrate]) at 72°C for 5 minutes, followed by hybridization performed at 37°C for 2 days by using a DNA probe for detecting HER2 gene and a probe for detecting human chromosome 17 centromere (PathVysion HER-2 DNA probe kit [manufactured by Abbott Japan Co., Ltd.]). After the hybridization, cell nuclei were stained for counting the number of cells by conducting the DAPI staining (2 **µ**g/ml PBS) at 25°C for 10 minutes. The amount of HER2 protein calculated by the IHC-DAB method, that is, a conventional tissue staining method, (IHC-DAB score) and the HER2 gene copy number calculated by the FISH method (FISH score) were compared with each other, resulting in finding that there was no quantitative relationship therebetween (Figure 8(a)). On the other hand, the number of fluorescent particles detected by using fluorescent particle-labeled trastuzumab calculated by the IHC-QDs method (IHC-QDs score) and the FISH score were compared with each other, resulting in finding that there was strong correlation between the IHC-QDs score and the FISH score (correlation coefficient (R) = 0.7009, p < 0.001) (Figure 8(b)). In other words, the amount of HER2 protein detected by trastuzumab calculated by the IHC-QDs method and the HER2 gene copy number were both quantitatively evaluated, so as to be correlated with each other. Although pathological determination on a case having IHC-DAB score 2 should be reexamined by the FISH method up to the present, IHC-QDs score calculated by the IHC-QDs method is thus in quantitative correlation with FISH score calculated by the FISH method, and hence, there is no need of reexamination by the FISH method. Furthermore, the IHC-QDs method is superior to the IHC-DAB method also because it can be conducted through more simplified operation procedures (Figure 9). Moreover, Figure 10 illustrates results of comparison between IHC-DAB scores and IHC-QDs scores. It is understood from Figure 10 that cases evaluated as the same criterion (of, for example, Score 2 or Score 3) by the IHC-DAB method may be detected in a very wide range when evaluated by obtaining IHC-QDs scores. Such results reveal the following: Although the IHC-DAB method and the IHC-QDs method are the same in terms of the immunohistostaining method, the conventional IHC-DAB method is an insufficient evaluation method because the expression level of HER2 protein can be evaluated in merely four stages by this method, but the IHC-QDs method can make highly accurate and quantitative evaluation by calculating IHC-QDs scores. Moreover, three cases surrounded with dotted lines in Figure 10 were evaluated as Score 2 by the IHC-DAB method but were evaluated as extremely low values by the IHC-QDs method, and the values substantially corresponded to cases of Score 0 or Score 1 of the IHC-DAB method. There is a possibility that such a difference is caused because the epitope is different between the anti-HER2 antibody (trastuzumab) used as a therapeutic agent in the IHC-QDs method and the anti-HER2 antibody used as a diagnostic agent in the IHC-DAB method. Accordingly, the diagnosis method employing the tissue staining using an antibody contained in a therapeutic agent is probably more effective for selecting patients eligible for administration of the therapeutic agent than a conventional diagnosis method, such as the IHC-DAB method, using an antibody different in the epitope from the antibody included in the therapeutic agent.

### Example 7

### [Comparison between therapeutic effect attained in single-agent treatment cases with trastuzumab and IHC-QDs score or FISH score]

As indexes corresponding to a therapeutic effect attained in single-agent treatment with trastuzumab, two indexes of Response (a therapeutic effect of the agent) and TTP (time to progression: time period when tumor growth is controlled by the administration of the agent) are widely used all over the world. It was verified whether or not the amount of HER2 protein detected by using trastuzumab calculated by the IHC-QDs method (IHC-QDs score) could be an index corresponding to the therapeutic effect apart from these two indexes. With respect to six cases shown in Table 2 below in which distance metastasis was observed and the single-agent treatment with trastuzumab was conducted, IHC-QDs scores were calculated so as to check the correlation with TTP. As a result, there was strong correlation between the IHC-QDs scores and the TTP (correlation coefficient (R) = 0.69) (Figure 11(b)). On the other hand, no correlation was found between FISH scores and the TTP (Figure 11(a)). These results reveal that a therapeutic effect attained by trastuzumab targeting HER2 in breast cancer tissues can be estimated by the IHC-QDs method using trastuzumab labeled with the quantum dot fluorescent particles, and that it is difficult to estimate, by the conventional FISH method, the therapeutic effect attained by trastuzumab.

**[Table 2]**

| No. | Age | Stage | ER/PgR | Metastatic site | FISH | IHC-DAB | IHC-QD | Response † | TTP (month)‡ |
|---|---|---|---|---|---|---|---|---|---|
| Case 32 | 60 | IIB | -/- | Liver | 4.25 | 3 | 8.17 | SD | 12 |
| Case 33 | 31 | I | -/- | Lung | 4.53 | 3 | 8.26 | PD | 3 |
| Case 34 | 74 | IIIC | -/- | Liver | 6.90 | 2 | 9.69 | PD | 3 |
| Case 35 | 32 | IIB | +/+ | Lung | 2.55 | 3 | 10.26 | SD | 27 |
| Case 36 | 73 | IIIA | -/- | Liver | 4.13 | 3 | 11.32 | PR | 50 |
| Case 37 | 36 | IIB | -/- | Bone | 7.90 | 3 | 12.17 | SD | 24 |

Abbreviated terms "PR", "PD" and "SD" referring to a response (a therapeutic effect of an agent) and a term "TTP" have the following meanings (see Manual for residents involved in medical care for cancer, third edition, published in September 2003):
Partial response (PR): at least a 30% decrease of cancer tissue in four weeks
Progressive disease (PD): at least a 20% increase of cancer tissue
Stable disease (SD): cancer tissue satisfying neither PR criterion nor PD criterion
Time to progression (TTP): time period when no progression is made or when therapeutic effect lasts

### Industrial Applicability

The determination method of the present invention is suitably employed in the fields of selection of patients eligible for administration of a medicine or estimation of a therapeutic effect of a medicine. For example, trastuzumab, that is, an antibody medicine for breast cancer, is used as an antibody labeled with quantum dot fluorescent particles, the number of fluorescent particles per cell detected by trastuzumab is obtained, and the thus obtained number may be used as an index for selecting patients eligible for administration of trastuzumab or estimating a therapeutic effect attained by trastuzumab.

## Claims

1. A method for determining effectiveness of a medicine containing an antibody as a component by employing a tissue staining method comprising the steps of:
(a) labeling an antibody in the medicine containing an antibody as a component with a fluorescent material and contacting the fluorescence-labeled antibody with a tissue sample;
(b) obtaining a fluorescence image by irradiating, with excitation light, a tissue site contacted with the antibody;
(c) obtaining an autofluorescence image in the same field of view and at the same focus as in the fluorescence image in a close region on a shorter wavelength side or a longer wavelength side of an acquisition wavelength region of fluorescence emitted by the fluorescent material; and
(d) obtaining a corrected fluorescence image by performing image processing for removing fluorescence brightness of the autofluorescence image from fluorescence brightness of the fluorescence image.

2. The method according to claim 1, wherein the antibody in the medicine containing an antibody as a component is an antibody recognizing a growth regulatory factor or a metastasis regulatory factor of cancer.

3. The method according to claim 2, wherein the growth regulatory factor or the metastasis regulatory factor of cancer is human epidermal growth factor receptor 2 (HER2).

4. The method according to claim 2 or 3, wherein the cancer is breast cancer.

5. The method according to any one of claims 1 to 4, wherein a difference in wavelength between the acquisition wavelength region of fluorescence emitted by the fluorescent material and the close region is 100 nm or less.

6. The method according to any one of claims 1 to 5, wherein the fluorescent material is a fluorescent particle.

7. The method according to claim 6, wherein the fluorescent particle is a quantum dot fluorescent particle.

8. The method according to claim 6 or 7, further comprising the steps of:
(e) counting a number of cells in the tissue site contacted with the antibody;
(f) identifying a single fluorescent particle on the basis of the fluorescence image and measuring average fluorescence brightness per fluorescent particle within the corrected fluorescence image corresponding to the single fluorescent particle; and
(g) obtaining a number of fluorescent particles by dividing total fluorescence brightness within the corrected fluorescence image by the average fluorescence brightness per fluorescent particle, and calculating a number of fluorescent particles per cell by dividing the number of fluorescent particles by the number of cells counted in step (e).

9. The method according to claim 8, wherein the single fluorescent particle is specified by utilizing a blinking property of the fluorescent particle.

## Patentansprüche

1. Verfahren zur Bestimmung der Wirksamkeit eines Arzneimittels, das einen Antikörper als eine Komponente enthält, durch Einsatz eines Gewebefärbungsverfahrens, das folgende Schritte umfasst:
(a) Markieren eines Antikörpers in dem Arzneimittel, das einen Antikörper als eine Komponente enthält, mit einer fluoreszierenden Substanz und Inkontaktbringen des fluoreszenzmarkierten Antikörpers mit einer Gewebeprobe;
(b) Erhalten eines Fluoreszenzbilds, indem eine mit dem Antikörper in Kontakt gebrachte Gewebestelle mit Anregungslicht bestrahlt wird;
(c) Erhalten eines Autofluoreszenzbilds in demselben Sichtfeld und an demselben Brennpunkt wie in dem Fluoreszenzbild in einem nahen Bereich auf der Seite der kürzeren Wellenlängen oder auf der Seite der längeren Wellenlängen eines Aufnahmewellenlängenbereichs von der Fluoreszenz, die von der fluoreszierenden Substanz emittiert wird; und
(d) Erhalten eines korrigierten Fluoreszenzbilds mittels Durchführen von Bildverarbeitung zur Beseitigung der Fluoreszenzhelligkeit des Autofluoreszenzbilds aus der Fluoreszenzhelligkeit des Fluoreszenzbilds.

2. Verfahren nach Anspruch 1, wobei der Antikörper in dem Arzneimittel, das einen Antikörper als eine Komponente enthält, ein Antikörper ist, der einen Wachstumsregulationsfaktor oder einen Metastasierungsregulationsfaktor von Krebs erkennt.

3. Verfahren nach Anspruch 2, wobei der Wachstumsregulationsfaktor oder der Metastasierungsregulationsfaktor von Krebs humaner epidermaler-Wachstumsfaktor-Rezeptor 2 (HER2) ist.

4. Verfahren nach Anspruch 2 oder 3, wobei der Krebs Brustkrebs ist.

5. Verfahren nach einem der Ansprüche 1 bis 4, wobei ein Unterschied in der Wellenlänge zwischen dem Aufnahmewellenlängenbereich von Fluoreszenz, die von der fluoreszierenden Substanz emittiert wird, und dem nahen Bereich 100 nm oder weniger beträgt.

6. Verfahren nach einem der Ansprüche 1 bis 5, wobei die fluoreszierende Substanz ein fluoreszierendes Teilchen ist.

7. Verfahren nach Anspruch 6, wobei das fluoreszierende Teilchen ein fluoreszierendes Quantenpunkt-Teilchen ist.

8. Verfahren nach Anspruch 6 oder 7, das weiterhin folgende Schritte umfasst:
(e) Zählen einer Anzahl von Zellen an der Gewebestelle, die mit dem Antikörper in Kontakt gebracht wurde;
(f) Identifizieren eines einzelnen fluoreszierenden Teilchens auf der Basis des Fluoreszenzbilds und Messen der durchschnittlichen Fluoreszenzhelligkeit pro fluoreszierendem Teilchen in dem korrigierten Fluoreszenzbild entsprechend dem einzelnen fluoreszierenden Teilchen; und
(g) Erhalten einer Anzahl von fluoreszierenden Teilchen, indem die Gesamt-Fluoreszenzhelligkeit in dem korrigierten Fluoreszenzbild durch die durchschnittliche Fluoreszenzhelligkeit pro fluoreszierendem Teilchen dividiert und eine Anzahl an fluoreszierenden Teilchen pro Zelle mittels Dividieren der Anzahl an fluoreszierenden Teilchen durch die Anzahl der in Schritt (e) gezählten Zellen berechnet wird.

9. Verfahren nach Anspruch 8, wobei das einzelne fluoreszierende Teilchen durch Verwendung einer Blinkeigenschaft des fluoreszierenden Teilchens angegeben wird.

## Revendications

1. Procédé permettant de déterminer l'efficacité d'un médicament contenant, comme composant, un anticorps en utilisant la méthode de coloration de tissu, comprenant les étapes consistant à :
(a) marquer un anticorps dans le médicament contenant, comme composant, un anticorps avec un matériau fluorescent et mettre l'anticorps marqué par fluorescence en contact avec un échantillon de tissu ;
(b) obtenir une image de fluorescence en irradiant, avec une lumière d'excitation, un site tissulaire mis en contact avec l'anticorps ;
(c) obtenir une image d'autofluorescence dans le même champ de vue et à la même distance focale que ceux de l'image de fluorescence dans une région proche des longueurs d'onde les plus courtes ou des longueurs d'onde les plus longues de la région des longueurs d'onde d'acquisition de la fluorescence émise par le matériau fluorescent ; et
(d) obtenir une image de fluorescence corrigée en effectuant un traitement d'image pour soustraire la brillance de fluorescence de l'image d'autofluorescence de la brillance de fluorescence de l'image de fluorescence.

2. Procédé selon la revendication 1, dans lequel l'anticorps du médicament contenant, comme composant, un anticorps est un anticorps reconnaissant un facteur de régulation de la croissance ou un facteur de régulation de la métastase d'un cancer.

3. Procédé selon la revendication 2, dans lequel le facteur de régulation de la croissance ou le facteur de régulation de la métastase d'un cancer est le récepteur du facteur de croissance épidermique humain 2 (HER2).

4. Procédé selon les revendications 2 ou 3, dans lequel le cancer est le cancer du sein.

5. Procédé selon l'une quelconque des revendications 1 à 4, dans lequel la différence de longueurs d'onde entre la région des longueurs d'onde d'acquisition de la fluorescence émise par le matériau fluorescent et la région proche est de 100 nm ou moins.

6. Procédé selon l'une quelconque des revendications 1 à 5, dans lequel le matériau fluorescent est une particule fluorescente.

7. Procédé selon la revendication 6, dans lequel la particule fluorescente est une particule fluorescente constituée de points quantiques.

8. Procédé selon la revendication 6 ou 7, comprenant en outre les étapes consistant à :
(e) compter un nombre de cellules dans le site tissulaire mis en contact avec l'anticorps ;
(f) identifier une particule fluorescente isolée d'après l'image de fluorescence et mesurer la brillance moyenne de fluorescence par particule fluorescente dans une image de fluorescence corrigée correspondant à la particule fluorescente isolée ; et
(g) obtenir un nombre de particules fluorescentes en divisant la brillance de fluorescence totale de l'image de fluorescence corrigée par la brillance de fluorescence moyenne par particule fluorescente, et calculer un nombre de particules fluorescentes par cellule en divisant le nombre de particules fluorescentes par le nombre de cellules comptées à l'étape (e).

9. Procédé selon la revendication 8, dans lequel la particule fluorescente isolée est définie en utilisant le clignotement de la particule fluorescente.
